# EUROPÄISCHE PATENTSCHRIFT

(11) **EP 1 181 321 B1**
(45) Veröffentlichungstag und Bekanntmachung des Hinweises auf die Patenterteilung: **28.07.2004**
(21) Anmeldenummer: 00920542.8
(22) Anmeldetag: 23.03.2000
(51) Int. Cl.: C08F 6/00, B01D 53/00, B01D 53/22

(54) **VERFAHREN ZUR ISOLIERUNG VON OLEFINEN AUS POLYOLEFINANLAGEN**
METHOD FOR ISOLATING OLEFINS FROM POLYOLEFIN PLANTS
PROCEDE D'ISOLEMENT D'OLEFINES PROVENANT D'INSTALLATIONS DE PRODUCTION DE POLYOLEFINES

(30) Priorität: 01.04.1999 DE 19915106
(43) Veröffentlichungstag der Anmeldung: 27.02.2002
(73) Patentinhaber: Basell Polyolefine GmbH, 50389 Wesseling (DE)
(72) Erfinder: FEINDT, Hans-Jacob, Flemington, NJ 08822-6930 (US); BITTERLICH, Stefan, D-67246 Dirmstein (DE); EVERTZ, Kaspar, D-67105 Schifferstadt (DE); HECKER, Manfred, D-53577 Neustadt (DE)
(86) Internationale Anmeldenummer: PCT/EP2000/002554
(87) Internationale Veröffentlichungsnummer: WO 2000/059957

(56) Entgegenhaltungen:
- EP-A- 0 646 603
- WO-A-99/45035
- DE-B- 1 520 498
- US-A- 4 588 790
- PATENT ABSTRACTS OF JAPAN vol. 1996, no. 10, 31. Oktober 1996 (1996-10-31) & JP 08 151413 A (UBE IND LTD), 11. Juni 1996 (1996-06-11)

## Beschreibung

Die vorliegende Erfindung betrifft ein Verfahren zur Isolierung von Olefinen aus Polyolefinanlagen sowie ein Verfahren und eine Vorrichtung zur Herstellung von Polyolefinen.

Die Gasphasenpolymerisation ist eine wichtige Technologie zur Herstellung von Polyolefinen. Der Katalysator, der meist in geträgerter Form eingesetzt wird, liegt dabei in Form von kleinen, rieselfähigen Partikeln vor, die als die Ausgangspunkte für die Polymerisation dienen. In der Regel wird die Gasphasenpolymerisation als Wirbelschichtverfahren durchgerührt. Dabei liegen die Katalysatorpartikel während der Polymerisation in einer Wirbelschicht vor. Die Monomere werden in der Regel mit dem Trägergas in die entsprechende Polyolefinanlage, die bevorzugt als Wirbelschicht-Polymerisationsreaktor ausgebildet ist, eingeführt. Katalysatorpartikeln werden an einer anderen Stelle des Polymerisationsreaktors, meist zusammen mit Inertgas, hinzugeführt. Als Katalysatoren eignen sich insbesondere Philipps- (auf der Basis von Chrom/Kieselgel), Ziegler- oder Metallocenkatalysatoren. Die Hinzugabe von Inertgas ist deshalb notwendig, da die zugrundeliegende heterogen katalysierte Polymerisation meist feuchtigkeits- und/oder sauerstoffempfindlich ist. Das resultierende Polymer fällt in fester Form an, so dass Katalysator und Polymer nebeneinander in der Wirbelschicht vorliegen. Nicht umgesetztes Monomer wird meist im Kreislauf geführt und übernimmt dabei den Abtransport von Reaktionswärme.

Gasphasenpolymerisationen werden beispielsweise dazu verwendet, um Polyethylen oder Polypropylen herzustellen. Es können aber auch durch dieses Verfahren Polymere hergestellt werden, die sich aus mehreren verschiedenen Monomereinheiten zusammensetzen, sogenannte Copolymere. Bei den Katalysatoren, die sich für die Gasphasenpolymerisation eignen, kommen wie einleitend beschrieben insbesondere Ziegler-, Philipps- und Metallocen-Katalysatoren in Frage. Philipps- sowie Metallocen-Katalysatoren sind sehr empfindlich gegenüber Katalysatorgiften, die selbst in geringen Konzentrationen (im ppm-Bereich) die Polymerisation unterbinden können. Solche Katalysatorgifte sind beispielsweise Schwefelverbindungen wie Schwefeldioxid oder Schwefelwasserstoff. Häufig treten diese in so geringen Konzentrationen auf, dass sie aus der gasförmigen Reaktionsmischung nicht unmittelbar nachweisbar sind. Es ist daher häufig schwer zu beurteilen, welche Katalysatorgifte für die Inaktivität eines Katalysators verantwortlich sind. Da sich beim kontinuierlichen Betrieb von Gasphasenpolymerisationsanlagen in der Regel Katalysatorgifte anreichern, ist es notwendig, einen Teil der gasförmigen Reaktionsmischung während des Betriebs als Abgas aus dem Reaktor zu entfernen.

Dabei besteht der Nachteil, dass auch Olefine als wertvolle Ausgangsstoffe verlorengehen, wenn das Abgas nicht aufgearbeitet wird. Das Abgas bei Gasphasenpolymerisationen enthält in der Regel vorwiegend Inertgas und nicht umgesetztes Olefin. Wird Inertgas nicht zum Teil aus dem Reaktor abgeführt, reichert sich dieses an, da es bei der Polymerisation nicht umgesetzt wird. Aus diesem Grund ist es notwendig, dem Reaktor, bei kontinuierlicher Betriebsweise ständig einen konstanten Abgasstrom zu entnehmen. In der Regel wird dabei wertvolles Olefin zusammen mit Inertgas und anderen Nebenkomponenten, wie z.B. Katalysatorgiften, abgeführt, anschließend das in dem abgeführten Gasgemisch enthaltende Olefin verbrannt und schließlich das erhaltene Verbrennungsgas verworfen. Der Verlust an Olefin ist finanziell beträchtlich, so dass versucht wurde, das Olefin zurückzugewinnen und dem Polymerisationsreaktor wieder zurückzuführen.

Die Isolierung des Olefins aus dem Abgas kann gemäß der US-A-5,521,264 durch Extraktion des Olefins realisiert werden, wobei in einem nächsten Schritt das Olefin von dem Extraktionsmittel getrennt werden muß. Diese Abtrennung ist apparativ recht aufwendig, so daß es in der Regel kostengünstiger ist, das Olefin nicht abzutrennen und es (nach der Verbrennung) mit dem Abgas zu verwerfen.

Der vorliegenden Erfindung liegt die Aufgabe zugrunde, das vorstehende Verfahren zur Herstellung von Polyolefinen so zu verbessern, daß die im Abgas enthaltenen Olefine zurückgewonnen und anschließend wieder in den Polymerisationsreaktor zurückgeführt werden können. Dabei ist von besonderer Bedeutung, daß das rückgeführte Olefin sowohl von Inertgasen als auch von Nebenprodukten, insbesondere Katalysatorgiften, befreit wird. Dabei sollte der apparative Aufwand möglichst gering gehalten werden, so daß die Rückgewinnung der Olefine wirtschaftlich rentabel ist.

Gelöst wird diese Aufgabe durch ein Verfahren zur Isolierung von Olefinen aus einem ein oder mehrere Olefine, Inertgas und Katalysatorgift enthaltenden Gasgemisch, wobei das Gasgemisch einer Trenneinheit zugeführt und in Olefin und Inertgas aufgetrennt wird. Das erfindungsgemäße Verfahren ist dann, dadurch gekennzeichnet, daß der Trenneinheit eine Apparatur vor- oder nachgeschaltet wird und daß
i) bei vorgeschalteter Apparatur das Katalysatorgift zumindest teilweise aus dem Gasgemisch und
ii) bei nachgeschalteter Apparatur das Katalysatorgift zumindest teilweise aus dem abgetrennten Olefin
entfernt wird.

Gemäß einer bevorzugten Ausführungsform der Erfindung fällt das Gasgemisch als Abgas bei der katalytischen Gasphasenpolymerisation von Olefinen an.

Das erfindungsgemäße Verfahren ist besonders geeignet, wenn Metallocen- oder Chrom/Kieselgel-Katalysatoren für die Polymerisation verwendet werden, da diese Katalysatoren besonders empfindlich gegenüber Katalysatorgiften sind. Häufig lassen sich diese Katalysatorgifte kaum identifizieren, so dass es von großer Bedeutung ist, selbst kleinste Mengen an Verunreinigungen, sogar im ppm-Bereich, möglichst vollständig zu entfernen. In der Regel ist der Anteil des Katalysatorgifts im Gasgemisch kleiner als 10⁻³ Gew.-%.

Zur Entfernung des Katalysatorgifts eignet sich erfindungsgemäß insbesondere eine Apparatur, die zur Stofftrennung ausgebildet ist. Als Apparatur zur Stofftrennung sollen Einrichtungen verstanden werden, die ein oder mehrere Molsiebe enthalten, welche Katalysatorgift adsorbieren, absorbieren oder chemisch binden können. Dabei kann die Einrichtung beispielsweise Molsiebe wie Aktivkohle, Zeolithe oder Waschflüssigkeit enthalten. Als entsprechendes Adsorptionsverfahren kommt insbesondere eine Druckwechseladsorption (pressure swing adsorption = PSA-Verfahren) in Frage. Prinzipiell eigenen sich aber auch Apparaturen, mit deren Hilfe Katalysatorgifte chemisch umgesetzt werden, wobei die entsprechenden Folgeprodukte der Katalysatorgifte selbst nicht mehr als Katalysatorgift fungieren können - solche Apparaturen können dann diese Folgeprodukte wieder (in den Gasstrom) freisetzen. Eine nach dem Prinzip der Tieftemperaturzerlegung arbeitende Kondensationseinrichtung kommt ebenso als Apparatur in Frage, wobei die Abtrennung des Katalysatorgifts in dieser Ausführungsform durch fraktionierte Kondensation erfolgt.

In der erfindungsgemäß besonders bevorzugten Betriebsvariante der Apparatur zur Stofftrennung nach dem Adsorptionsverfahren liegt die Betriebstemperatur in der Beladungsphase im Bereich von -30 bis 100 °C, vorzugsweise im Bereich von 10 bis 50 °C, während der Druck in dieser Phase im Bereich von 3 bis 50 bar liegt, vorzugsweise von 10 bis 30 bar. Bei einem Betrieb mit periodischem Wechsel des Adsorbens oder mit periodischer Regenerierung des Adsorbens, beispiesweise mittels Stickstoff, liegen die optimalen Temperaturen zum Betrieb der Apparatur erfindungsgemäß im Bereich von 80 bis 240 °C.

Das bei dem Polymerisationsverfahren anfallende Abgas besteht in der Regel im wesentlichen (bevorzugt zu mehr als 95 %) aus Inertgas und Olefin. Dieses Gasgemisch enthält in der Regel 20 bis 80 Vol.-%, bevorzugt 40 bis 60 Vol.-% Olefin. Die Isolierung des Olefins von dem Inertgas erfolgt mit einer Trenneinheit, die beispielsweise eine Membraneinrichtung ist, die eine oder mehrere Membranen aufweist, oder eine Destillationseinrichtung. Solche Membraneinrichtungen zur Rückgewinnung von Olefinen enthalten zumeist eine oder mehrere Diffusionsmembranen, die Inertgase wie z.B. Stickstoff zurückhalten und für betreffende Olefine (allgemein für viele organische Verbindungen, insbesondere für zahlreiche Kohlenwasserstoffe) durchlässig sind [M. Jacobs, D. Gottschlich, K. Kaschemekat, Membrane Technology & Research, Inc., March 10, 1998]. In aller Regel erfolgt dabei die Auftrennung in Olefin und Inertgas nicht vollständig, so dass sowohl das abgetrennte Olefin noch Inertgas(reste) als auch das abgetrennte Inertgas noch Olefin(reste), enthalten kann. In diesem Zusammenhang kann man auch von der Anreicherung der entsprechenden Komponenten sprechen. In Frage kommende Trenneinheiten eignen sich prinzipiell nicht zur Entfernung oder Abtrennung von Katalysatorgiften - diese Funktion wird erfindungsgemäß durch eine entsprechende Apparatur wahrgenommen. So können beispielsweise als Trenneinheit eingesetzte Membraneinrichtungen nicht zur Entfernung oder Abtrennung von Katalysatorgift verwendet werden.

Erfindungsgemäß bevorzugte Membranen sind sogenannte Compositmembranen, die im wesentlichen aus einer dichten Polymerschicht, beispielsweise aus einem Polydialkylsiloxan wie Polydimethylsiloxan oder Polyoctylmethylsiloxan, auf einem porösen Trägermaterial bestehen. Als Trägermaterialien kommen Polymere wie Polyester, Polystyrole, Polyamide oder auch Polyolefine in Frage. Die vorstehend beschriebenen Membranen werden erfindungsgemäß in Form von Spiralwickel- Flach- oder auch Kissenmodulen eingesetzt.

Die Membrantrennung wird vorzugsweise bei Temperaturen im Bereich von -30 bis 100 °C, besonders bevorzugt von 0 bis 50 °C, durchgeführt, wobei auf der Zufuhrseite zweckmäßigerweise ein Druck in Bereich von 3 bis 50 bar anliegt, vorzugsweise von 10 bis 30 bar, während der Druck auf der Permeatseite einen Wert von 0,1 bis 10 bar, vorzugsweise von 0,8 bis 5 bar, einnimmt.

Erfindungsgemäß werden insbesondere Ethylen oder Propylen als Olefin eingesetzt. Es können jedoch auch verschiedene Olefine zugleich polymerisiert werden, so dass Copolymere erhalten werden. Prinzipiell können erfindungsgemäß alle Olefine eingesetzt werden, die polymerisiert oder copolymerisiert werden können. Da katalytische Gasphasenpolymerisationen in der Regel stark sauerstoff und/oder feuchtigkeitsempfindlich sind, werden diese meist in einer Inertgasatmosphäre durchgeführt. Als Inertgas eignet sich insbesondere Stickstoff. Prinzipiell können jedoch alle bei dem zugrundeliegenden Herstellungsverfahren inert wirkenden Gase oder Gasgemische, z.B. Alkane, als Inertgas eingesetzt werden. Als Katalysatorgift wird vorzugsweise Schwefeldioxid abgetrennt.

Außerdem wird eine Vorrichtung zur Durchführung des erfindungsgemäßen Verfahrens bereitgestellt, wobei diese folgende Einrichtungen enthält:
a) einen Wirbelschicht-Polymerisationsreaktor,
b) einen Entladungstank,
c) ein Filter für die Rückhaltung feiner Polymerpartikel,
d) eine Apparatur zur Entfernung des Katalysatorgifts,
e) eine Trenneinheit zur Trennung von Olefin und Inertgas,
f) einen Kompressor zur Rückführung des isolierten Olefins in den Wirbelschicht-Polymerisationsreaktor
sowie Verbindungsleitungen zwischen den Einrichtungen a) bis f).

Im folgenden wird die Erfindung beispielhaft anhand der in der anliegenden Zeichnung dargestellten einzigen Figur näher erläutert.

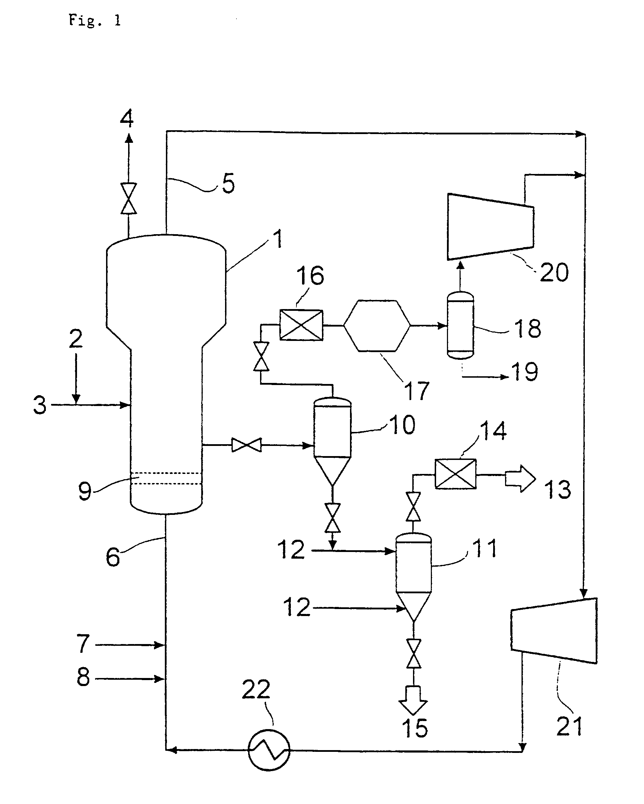

Fig. 1 zeigt ein Fließschema einer Polyolefinanlage mit einer Vorrichtung zur Isolierung von Polyolefinen.

In der in Fig. 1 gezeigten Polyolefinanlage findet die Gasphasenpolymerisation in einem Polymerisationsreaktor 1 statt. Der Katalysator wird zusammen mit dem Inertgas in den Polymerisationsreaktor 1 eingeführt, wobei der Katalysator in Form von Partikeln an der Zufuhrstelle 2 und das Inertgas an der Zufuhrstelle 3 hinzugegeben werden. Im oberen Bereich des Polymerisationsreaktors 1 befindet sich eine Abgasleitung 4 und das Ableitungsrohr 5 für Gas, das dem Reaktor als Kreisgas wieder zugeführt wird. Unterhalb des Polymerisationsreaktors 1 befindet sich das Einleitungsrohr für Wirbelgas 6, dem an der Zufuhrstelle 7 Inertgas und an der Zufuhrstelle 8 gasförmiges Monomer zugeführt wird. In den unteren Bereich des Polymerisationsreaktors 1 eingeführtes Wirbelgas wird an einem Gasverteilerboden 9 verteilt, so dass sich im Polymerisationsreaktor 1 eine stabile Wirbelschicht ausbildet.

Das sich im Polymerisationsreaktor 1 bildende Polyolefin wird zusammen mit den im Polymerisationsreaktor 1 enthaltenen Gasen in den Entladungstank 10 geführt. In dem Entladungstank 10 werden die Gase von dem Polymer getrennt. Das Polymer wird in einen Reinigungstank 11 überführt, wobei zur Reinigung von den Zufuhrstellen 12 Inertgas in den Reinigungstank 11 eingeblasen wird. Das im Reinigungstank 11 anfallende, vorwiegend Inertgas enthaltende Gas wird, bevor es an der Ablaßstelle 13 als Abgas freigesetzt wird, durch einen Filter 14 zur Rückhaltung feiner Polymerpartikel geleitet. Am Ablaß 15 fällt das im Polymerisationsreaktor 1 gebildete Polymer an.

Das im Entladungstank 10 von dem Polymer abgetrennten Gasgemisch wird in einem weiteren Filter 16 von feinen Polymerpartikeln befreit. Anschließend wird das Gasgemisch durch eine Apparatur 17 zur Entfernung von Katalysatorgiften , z.B. eine Einrichtung, die ein oder mehrere Molsiebe enthält, geleitet. Das von Katalysatorgiften gereinigte Gasgemisch wird in einer Trenneinheit 18 (z.B. einer Membraneinrichtung) in Inertgas und Olefin aufgetrennt. Diese Trennung erfolgt jedoch nicht vollständig, so dass das abgetrennte Olefin noch Inertgas(reste) und das abgetrennte Inertgas noch Olefin(reste) enthält. Das Inertgas wird durch das Ableitungsrohr 19 abgeführt und kann anschließend noch als Stripgas (bevorzugt im gleichen Prozeß) eingesetzt werden, wobei das Inertgas als Stripgas über nicht dargestellte Leitungen in den Entladungstank 10 und/oder in den Reinigungstank 11 (z.B. über die Zufuhrstellen 12) eingeleitet werden kann.

Die isolierten Olefine werden mit Hilfe eines Gaskompressors 20 in den Kreisgasstrom eingeleitet, in dem auch das durch das Ableitungsrohr 5 abgeführte Reaktorgas geführt wird. Das Kreisgas wird mittels eines zweiten Gaskompressors 21 durch einen Wärmetauscher 22 geleitet, in dem das heiße Gas abgekühlt und anschließend dem Polymerisationsreaktor 1 als Wirbelgas bereitgestellt wird.

In einer weiteren Ausführungsform der Erfindung kann die Apparatur 17 zur Entfernung des Katalysatorgifts der Trenneinheit 18 (Trennung von Olefinen und Inertgasen) nachgeschaltet sein. In einem solchen in der Figur nicht dargestellten Fall wird ausschließlich das abgetrennte Olefin durch die Apparatur 17 zur Entfernung des Katalysatorgifts hindurchgeleitet.

Das erfindungsgemäße Verfahren zur Isolierung von Olefinen aus Polyolefinanlagen kann nicht nur speziell bei der Gasphasenpolymerisation, sondern prinzipiell bei allen für die Polymerisation von Polyolefinen geeigneten Polymerisationstechniken, wie z.B. Lösung- oder Suspensionspolymerisation, angewendet werden. Es arbeitet unter den vorstehend ausführlich angegebenen Bedingungen zuverlässig, effekitv und unter wirtschaftlichen Bedingungen.

## Patentansprüche

1. Verfahren zur Isolierung von Olefinen aus einem ein oder mehrere Olefine, Inertgas und Katalysatorgift enthaltenden Gasgemisch, wobei das Gasgemisch einer Trenneinheit (18) zugeführt und in Olefin und Inertgas aufgetrennt wird, **dadurch gekennzeichnet, dass** der Trenneinheit (18) eine Apparatur (17) vor- oder nachgeschaltet ist und dass
i) bei vorgeschalteter Apparatur (17) das Katalysatorgift zumindest teilweise aus dem Gasgemisch und
ii) bei nachgeschalteter Apparatur (17) das Katalysatorgift zumindest teilweise aus dem abgetrennten Olefin
entfernt wird.

2. Verfahren nach Anspruch 1, **dadurch gekennzeichnet dass** das Gasgemisch als Abgas bei der katalytischen Gasphasenpolymerisation von Olefinen anfällt.

3. Verfahren nach Anspruch 1 oder 2, **dadurch gekennzeichnet, dass** als Trenneinheit (18) eine Membraneinrichtung, die eine oder mehrere Membranen enthält, oder eine Destillationseinrichtung eingesetzt wird.

4. Verfahren nach Anspruch 3, **dadurch gekennzeichnet, dass** als Trenneinheit (18) eine Membraneinrichtung eingesetzt wird, die als Spiral-, Wickel- oder Flachmodul ausgebildet ist, und dass die Trenneinheit (18) bei Betriebstemperaturen im Bereich von -30 bis 100 °C betrieben wird.

5. Verfahren nach einem der Ansprüche 1 bis 4, **dadurch gekennzeichnet, dass** als Apparatur (17) zur Entfernung des Katalysatorgifts eine Einrichtung enthaltend ein oder mehrere Molsiebe verwendet wird.

6. Verfahren nach einem der Ansprüche 1 bis 5, **dadurch gekennzeichnet, dass** als Olefin Ethylen oder Propylen eingesetzt wird.

7. Verfahren nach einem der Ansprüche 1 bis 6, **dadurch gekennzeichnet, dass** als Inertgas Stickstoff verwendet wird.

8. Verfahren nach einem der Ansprüche 1 bis 7, **dadurch gekennzeichnet, dass** der prozentuale Anteil des Katalysatorgifts im Gasgemisch kleiner/gleich 10⁻³ Gew.-% ist und dass als Katalysatorgift Schwefeldioxid aus dem Gasgemisch entfernt wird.

9. Verfahren nach einem der Ansprüche 1 bis 8, **dadurch gekennzeichnet, dass** das Gasgemisch im wesentlichen aus Inertgas und Olefin besteht und dabei das Gasgemisch 20 bis 80 Vol.-%, bevorzugt 40 bis 60 Vol.-% Olefin enthält.

10. Vorrichtung zur Durchführung des Verfahrens nach einem der Ansprüche 1 bis 9 enthaltend mindestens folgende Einrichtungen:
a) einen Polymerisationsreaktor (1),
b) einen Entladungstank (10),
c) ein Filter für die Rückhaltung feiner Polymerpartikel (14),
d) eine Apparatur zur Entfernung des Katalysatorgifts (17),
e) eine Trenneinheit (18) zur Trennung von Olefin und Inertgas,
f) einen Gaskompressor (20) zur Rückführung des isolierten Olefins in den Wirbelschicht-Polymerisationsreaktor (1)
und Verbindungsleitungen zwischen den Einrichtungen a) bis f).

## Claims

1. A process for isolating olefins from a gas mixture containing one or more olefins, inert gas and catalyst poison by steps which include the gas mixture being fed to a separation unit (18) and being separated into olefin and inert gas, **characterized in that** the separation unit (18) has connected to it an upstream or downstream apparatus (17) and **in that**
i) the catalyst poison is at least partially removed from the gas mixture in the case of an upstream apparatus (17) and
ii) the catalyst poison is at least partially removed from the separated-off olefin in the case of a downstream apparatus (17).

2. A process according to claim 1, **characterized in that** the gas mixture is obtained as an offgas in the catalytic gas phase polymerization of olefins.

3. A process according to claim 1 or 2, **characterized in that** the separation unit (18) is a membrane device, which contains one or more membranes, or a distillation device.

4. A process according to claim 3, **characterized in that** the separation unit (18) is a membrane device which is configured as a spiral, wound or flat module and **in that** the separation unit (18) is operated at operating temperatures in the range from -30°C to 100°C.

5. A process according to any one of claims 1 to 4, **characterized in that** the apparatus (17) for removing the catalyst poison is a device containing one or more molecular sieves.

6. A process according to any one of claims 1 to 5, **characterized in that** the olefin used is ethylene or propylene.

7. A process according to any one of claims 1 to 6, **characterized in that** the inert gas used is nitrogen.

8. A process according to any one of claims 1 to 7, **characterized in that** the percentage of catalyst poison in the gas mixture is not more than 10⁻³% by weight and **in that** the catalyst poison removed from the gas mixture is sulfur dioxide.

9. A process according to any one of claims 1 to 8, **characterized in that** the gas mixture consists essentially of inert gas and olefin and contains from 20% to 80% by volume and preferably from 40% to 60% by volume of olefin.

10. Equipment for carrying out the process according to any one of claims 1 to 9, containing at least the following devices:
a) a polymerization reactor (1),
b) a discharge tank (10),
c) a filter for retaining fine polymer particles (14),
d) an apparatus for removing the catalyst poison (17),
e) a separation unit (18) to separate olefin and inert gas,
f) a gas compressor (20) for recycling the isolated olefin into the fluidized bed polymerization reactor (1)
and connection lines between the devices a) to f).

## Revendications

1. Procédé d'isolement d'oléfines à partir d'un mélange de gaz contenant un ou plusieurs oléfine, gaz inerte ou poison catalytique, dans lequel le mélange de gaz alimente une unité de séparation (18) et est séparé en oléfine et gaz inerte, **caractérisé en ce que** l'unité de séparation (18) est disposée avant ou après un appareillage (17) et **en ce que**
i) quand l'appareillage (17) se trouve devant, le poison catalytique est éliminé au moins en partie du mélange de gaz, et
ii) quand l'appareillage (17) se trouve derrière, le poison catalytique est éliminé au moins en partie de l'oléfine séparée.

2. Procédé selon la revendication 1, **caractérisé en ce que** le mélange de gaz est produit en tant que gaz d'échappement pendant la polymérisation catalytique en phase gazeuse d'oléfines.

3. Procédé selon la revendication 1 ou 2, **caractérisé en ce qu'**en tant qu'unité de séparation (18), un dispositif à membrane qui contient une ou plusieurs membranes, ou un dispositif de distillation est installé.

4. Procédé selon la revendication 3, **caractérisé en ce qu'**en tant qu'unité de séparation (18), un dispositif à membrane qui est formé en tant que module spirale, pelote, ou plat est installé, et **en ce que** l'unité de séparation (18) est opérée à des températures de fonctionnement dans le domaine de -30 à 100°C.

5. Procédé selon l'une des revendications 1 à 4, **caractérisé en ce qu'**en tant qu'appareillage (17) d'élimination du poison catalytique, un dispositif contenant un ou plusieurs tamis moléculaires est utilisé.

6. Procédé selon l'une des revendications 1 à 5, **caractérisé en ce qu'**en tant qu'oléfine, l'éthylène ou le propylène est utilisé.

7. Procédé selon l'une des revendications 1 à 6, **caractérisé en ce que** l'azote est utilisé en tant que gaz inerte.

8. Procédé selon l'une des revendications 1 à 7, **caractérisé en ce que** la teneur proportionnelle en poison catalytique dans le mélange de gaz est inférieure/égale à 10⁻³% en poids et **en ce qu'**en tant que poison catalytique, le dioxyde de soufre est éliminé du mélange de gaz.

9. Procédé selon l'une des revendications 1 à 8, **caractérisé en ce que** le mélange de gaz se compose pour l'essentiel de gaz inerte et d'oléfine et par-là le mélange de gaz contient 20 à 80 % en volume, de préférence 40 à 60 % en volume d'oléfine.

10. Montage de réalisation du procédé selon l'une des revendications 1 à 9, contenant au moins les dispositifs suivants :
a) un réacteur de polymérisation (1),
b) un réservoir de décharge (10),
c) un filtre pour la rétention de particules de polymère fines (14),
d) un appareillage d'élimination du poison catalytique (17),
e) une unité de séparation (18) pour séparer l'oléfine et le gaz inerte,
f) un compresseur de gaz (20) pour rapatrier l'oléfine isolée dans le réacteur de polymérisation à lit fluidisé (1)
et des canalisations de liaison entre les dispositifs a) à f).
